(19)

**Europäisches Patentamt**
**European Patent Office**
**Office européen des brevets**

(11) **EP 3 325 048 B1**

(12) **EUROPEAN PATENT SPECIFICATION**

(45) Date of publication and mention
of the grant of the patent:
**04.10.2023 Bulletin 2023/40**

(21) Application number: **16728692.1**

(22) Date of filing: **13.06.2016**

(51) International Patent Classification (IPC):
**A61M 5/168** $^{(2006.01)}$     **A61M 5/145** $^{(2006.01)}$

(52) Cooperative Patent Classification (CPC):
**A61M 5/16827; A61M 5/1452**

(86) International application number:
**PCT/EP2016/063479**

(87) International publication number:
**WO 2017/012781 (26.01.2017 Gazette 2017/04)**

(54) **SYSTEM FOR PERFORMING A CONTINUOUS INFUSION PROCESS**

SYSTEM ZUR DURCHFÜHRUNG EINES KONTINUIERLICHEN INFUSIONSVERFAHREN

SYSTÈME POUR EFFECTUER UN PROCESSUS D'INFUSION CONTINUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.07.2015 PCT/EP2015/306177**

(43) Date of publication of application:
**30.05.2018 Bulletin 2018/22**

(73) Proprietor: **Fresenius Vial SAS
38590 Brézins (FR)**

(72) Inventors:
• **BURLAT, Maxime
42800 Genilac (FR)**

• **GUERRINI, Alexandre
38600 Fontaine (FR)**

(74) Representative: **Fresenius Kabi Deutschland
GmbH
Patent Department - MedTech
Else-Kröner-Straße 1
61352 Bad Homburg (DE)**

(56) References cited:
**WO-A1-2009/149367     WO-A1-2012/167090
WO-A2-01/80928          WO-A2-2005/084273
US-A1- 2006 224 140**

**Description**

The invention relates to a system for performing a continuous infusion process using at least two infusion devices.

In a first phase a first fluid from a first medication container is delivered through a first delivery line at a first flow rate using a first infusion device. After the first medication container has been depleted or nearly depleted, the infusion is taken over by a second infusion device so that in a second phase the second infusion device delivers a second fluid from a second medication container through a second delivery line at a second flow rate. The first delivery line and the second delivery line both are connected to a relay device, which is constituted to receive the first fluid via the first delivery line in the first phase and the second fluid via the second delivery line in the second phase. The relay device discharges the fluid it receives to an outlet delivery line, via which infusion into a patient may take place.

A method of this kind is for example known from US 2008/0269678 A1. Herein, two infusion devices in the shape of syringe pumps are applied in a concerted fashion in order to achieve a continuous infusion process, the infusion devices being connected via delivery lines to a coupler and via the coupler to a common infusion line. When a first medication container is depleted or nearly depleted, a controller automatically executes a relay function, sending control signals and instructing a first driver of the first infusion device to cease administration of a fluid from the first medication container and a second driver of the second infusion device to begin administration of a fluid from a second medication container in a closely-timed sequence.

A method of this kind may for example be used for the so called catecholamine infusion. Within the catecholamine infusion, medication such as adrenaline, noradrenaline or dobutamine may be administered to a patient for example for the purpose of resuscitation. Medication in this context is generally administered intravenously at a rather low dose rate, wherein it must be taken care that the dose rate is stable and continuous, because otherwise blood pressure and heart frequency peaks or drops may occur due to peaks or drops in the dose rate of the administered medication.

A system of multiple infusion devices may be used to continuously administer a medication to a patient over a comparatively long period of time at a constant dose rate. For this, however, it is necessary that the infusion devices are operated in a controlled, concerted fashion such that, upon depletion of a medication container associated with a first infusion device, infusion by a second infusion device from a second medication container starts in order to insure a continuous, non-interrupted infusion process.

In this regard, however, due to the length of the delivery lines connected to the infusion devices a second medication from a second infusion device may not immediately be delivered to a patient upon stopping the infusion by a first infusion device and starting the infusion by the second infusion device. This is due to the fact that during infusion by the first infusion device the outlet delivery line connecting the relay device with the patient is filled by the medication stemming from the first medication container associated with the first infusion device such that, upon starting infusion by the second infusion device, at first the residual medication from the first medication container in the outlet delivery line will be administered to the patient. This however may be problematic if the flow rate of the second infusion device is different than the flow rate of the first infusion device, leading to an increase or decrease in the dose rate by which the residual medication in the outlet delivery line is delivered to the patient.

There is, hence, a desire for a method and a system which reliably ensure that upon switching infusion from a first infusion device to a second infusion device a constant dose rate can be obtained.

Within a fluid infusion system as described in US 2011/0087189 A1 a fluid infusion pump discharges a fluid to a test subject in accordance with an infusion routine including for example a rate, a volume, a starter time, an end time and a duration.

In a system known from US 2013/0218080 A1 drugs are mixed with a carrier fluid using a multiplicity of infusion devices connected to a flow junction structure, via which the mixed fluid is delivered for example to a patient.

In a drug delivery system disclosed in WO 2005/084273 A2, a control means initially clears a first, or old, drug from the device based. After the old drug is cleared, then the control means automatically modifies the Current Profile data to match a second, or new, delivery profile for controlling delivery of a second, or new, drug.

According to a method for transitioning between delivery of therapeutic substances in US 2006/224140 A1, a bridge duration equal to the known volume divided by the known flow rate is calculated and delivery of a first and second therapeutic substance is controlled taking bridge duration into consideration.

In a hazardous fluid delivery system disclosed in WO 2009/149367 A2, a first fluid path element and second fluid path element fluidly are coupled together and a pump unit may be provided for dispensing fluid from the first and second fluid path elements optionally into a third fluid path element.

It is an object of the instant invention to provide a system for performing a continuous infusion process using at least two infusion devices, the system allowing for a continuous infusion at a desired, in particular constant dose rate in particular when switching from one infusion device to another.

This object is achieved by means of a method described in the following.

Accordingly, within the method the second infusion device is operated in an intermediate phase, subsequent to the delivery of the first fluid using the first infusion device in the first phase, for a predetermined delivery time to deliver the second fluid at the first flow rate, the predetermined delivery time being determined by taking the predefined delivery volume of the outlet delivery line into account.

The method starts from the finding that a residual volume of the first fluid remains in the outlet delivery line upon termination of the first phase, namely after terminating the first infusion device to deliver the first fluid from the first medication container through the first delivery line (for example due to the first medication container being depleted or being nearly depleted).

If in this case - with the residual first fluid being present in the outlet delivery line - the second infusion device takes over the infusion and would deliver the second fluid from the second medication container at the second flow rate, this would cause the residual first fluid in the outlet delivery line to be administered to the patient at the second flow rate, which may be different than the first flow rate. If the second flow rate for example is larger than the first flow rate, this would cause an increased dose rate by which the residual first fluid in the outlet delivery line is delivered to the patient. If in turn the second flow rate is smaller than the first flow rate, the residual first fluid in the outlet delivery line would be administered to the patient at a reduced dose rate.

In order to overcome a change in dose rate by which the residual first fluid in the outlet delivery line is administered to the patient, the second infusion device, upon taking over the infusion process, for a predefined delivery time is operated at the first flow rate, i.e., at the flow rate of the first infusion device. This causes the residual first fluid in the outlet delivery line to be administered to the patient at the same flow rate at which the first infusion device has been operated, such that no change in dose rate occurs when the second infusion device starts its infusion operation.

The second infusion device is operated at the first flow rate for the predetermined delivery time. The predetermined delivery time herein is determined by taking into account the delivery volume of the outlet delivery line, i.e., the volume of the line in which residual first fluid is present after termination of the infusion operation of the first infusion device. The delivery volume of the outlet delivery line indicates the fluid capacity of the outlet delivery line. At termination of the infusion by the first infusion device the outlet delivery line is (completely) filled with the first fluid delivered by the first infusion device. The predetermined delivery time hence is determined as that time that is necessary to deplete the outlet delivery line of the residual first fluid at the first flow rate.

In the intermediate phase the second infusion device hence is operated at the first flow rate, i.e., at the flow rate of the first infusion device. At the end of the intermediate phase, i.e., after the outlet delivery line has been depleted of the residual first fluid, the second infusion device is switched to the second flow rate by which the second fluid is to be delivered for administration to the patient.

The first fluid and the second fluid may for example comprise the same medication, but at a different concentration. In order to ensure a constant dose rate (the dose rate equals the flow rate times the concentration of medication in the fluid) throughout the entire infusion process, hence, the flow rates by which the different infusion devices are operated ought to be different. For example, if the concentration of the medication in the first fluid is four times larger than in the second fluid, the first flow rate is by a factor of four smaller than the second flow rate. Upon switching to the second infusion device, hence, the flow rate is increased in order to deliver the second fluid from the second medication container associated with the second infusion device at an increased flow rate, such that the same dose rate as during infusion by means of the first infusion device is achieved.

The invention comprises a system as defined in claim 1, i.e. a system for performing a continuous infusion process using at least two infusion devices. The system comprises: a first infusion device constituted to deliver a first fluid from a first medication container through a first delivery line at a first flow rate in a first phase; a second infusion device constituted to deliver a second fluid from a second medication container through a second delivery line at a second flow rate in a second phase following the first phase; and a relay device connected to said first delivery line and said second delivery line and constituted to receive the first fluid via the first delivery line in the first phase and the second fluid via the second delivery line in the second phase. The relay device is further constituted to discharge the received first or second fluid to an outlet delivery line connected to the relay device for administration to a patient, the outlet delivery line having a predefined delivery volume.

According to the proposed solution, the system is constituted such that, subsequently to the delivery of the first fluid using the first infusion device in the first phase, the second infusion device in an intermediate phase is operated for a predetermined delivery time to deliver the second fluid at the first flow rate, the predetermined delivery time being determined taking the predefined delivery volume of the outlet delivery line into account.

The advantages and advantageous embodiments described above for the method equally apply also to said system.

A method and a system of this kind are for example applicable for a catecholamine infusion in which substances belonging to the group of dopamines and its derivates are infused to a patient. A medication used in this regard can for example comprise dopamine, noradrenaline or adrenaline. The infusion of such medication can be used for example in the intensive care and emergency medicine for resuscitation of a patient or the like. The medication is infused intravenously to a patient, wherein a stable, steady infusion of the medication at a constant dose rate is desired in order to avoid peaks or drops of the dose rate, which otherwise may lead to peaks or drops in the blood pressure or the heart frequency of the patient.

In one embodiment, the first infusion device and the second infusion device each comprise a processor, wherein the first infusion device and the second infusion device are in communication connection with each other for controlling the operation of the second infusion device in dependence of the operation of the first infusion device. If for example the first medication container associated with the first infusion device is depleted or is nearly depleted, a control signal may be issued by one of the processors which controls the second infusion device to start its infusion operation, while another control signal is issued that stops the first infusion device. The second infusion operation hence is performed in a concerted fashion following the first infusion operation such that a continuous infusion of medication to the patient can be obtained.

The infusion devices are in communication connection with each other and may control each other to perform the continuous infusion operation. It however is also possible that an external control device is provided, which generates control signals and controls the operation of the first infusion device and the second infusion device.

In this regard it is to be noted that also more than two infusion devices could be present and can take part in the continuous infusion process. Hence, after the second medication container associated with the second infusion device is depleted or is nearly depleted the infusion operation can be continued by a third infusion device.

In this regard it further is to be noted that a continuous infusion operation involving more than two phases can also be obtained by reusing the infusion devices, for example by refilling or replacing the first medication container associated with the first infusion device after the first medication container has been emptied and after infusion has been taken over by the second infusion device.

To provide for a continuous infusion involving the multiple infusion devices, the infusion devices beneficially exchange information concerning for example their flow rates, the filling levels of their associated medication containers, their operational status such as start or stop signals, the type of fluids and their characteristics in their associated medication containers, wherein other or more information may be present and may be exchanged. One infusion device hence knows about the operational status of the other infusion device such that, according to the invention, the second infusion device may determine the predefined delivery time at which it shall be run in the intermediate phase following infusion by the first infusion device according to the first flow rate and the delivery volume of the outlet delivery line.

The delivery volume (also denoted as dead volume) of the outlet delivery line can for example be input by a user to one or multiple infusion devices and hence is known to the first and/or the second infusion device. Or it can be predefined for an infusion set connected to the infusion devices, such that by recognition of the infusion set the delivery volume of the outlet delivery line is known to the infusion devices. Or the delivery volume of the outlet delivery line can be measured in an initial calibration step by measuring the volume of fluid that can be received in the outlet delivery line. Alternatively, an analysis of the pressure in the delivery lines could be performed by the infusion devices in order to draw conclusions about the volume of the outlet delivery line.

The method and system as described above may in particular make use of infusion devices in the shape of syringe pumps receiving a syringe and acting onto the syringe for delivering medication from a cylindrical tube of the syringe. In principle, however, the method and system may also make use of infusion devices such as peristaltic (volumetric) infusion pumps or other infusion devices, such that the invention is not limited to the use of syringe pumps.

The invention shall subsequently be described in more detail with regard to the embodiments shown in the Figures. Herein:

Fig. 1    shows a schematic view of a system comprising a multiplicity of infusion devices used for continuously infusing medication towards a patient;

Fig. 2    shows the drawing of Fig. 1 in a first phase in which the infusion is carried out by a first infusion device;

Fig. 3    shows the schematic drawing at the end of the first phase after depletion of a medication container of the first infusion device;

Fig. 4    shows the schematic drawing in an intermediate phase in which the infusion has been taken over by a second infusion device;

Fig. 5    shows the schematic drawing in a second phase in which the infusion is carried out by the second infusion device; and

Fig. 6    shows a schematic flow diagram of the principle method steps for carrying out the continuous infusion process.

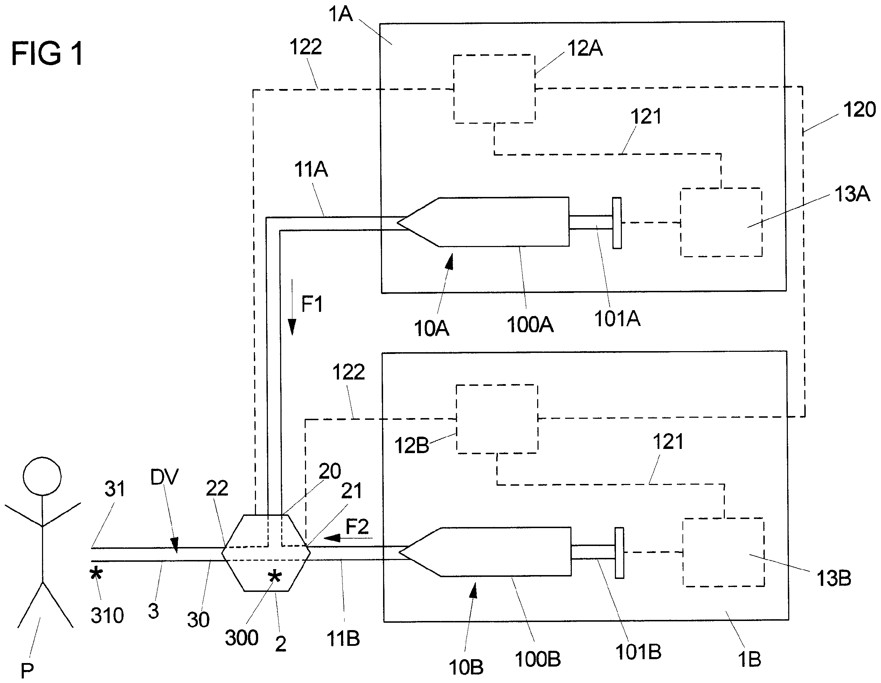

Fig. 1 shows a schematic drawing of a system comprising, in the shown embodiment, two infusion devices 1A, 1B which each comprise a pump device 10A, 10B in the shape of a syringe for delivering a fluid from a medication container 100A, 100B through a delivery line 11A, 11B towards a patient P. Each pump device 10A, 10B shaped as a syringe comprises a cylindrical tube constituting the medication container 100A, 100B and a plunger 101A, 101B received in the cylindrical tube. The pump device 10A, 10B is received for example in a suitable holding device of the associated infusion device 1A, 1B constituted as a syringe pump and acting onto the plunger 101A, 101B of the associated syringe by means of an electric drive device 13A, 13B for continuously pushing the plunger 101A, 101B into the cylindrical tube 100A, 100B to deliver the fluid received in the cylindrical tube 100A, 100B at a constant flow rate towards the patient P.

The system is set up to perform a continuous infusion operation. For this, the delivery lines 11A, 11B are each connected to a common communication node in the shape of a relay device 2. The delivery lines 11A, 11B are connected to inlets 20, 21 of the relay device 2. The relay device 2 is constituted to receive fluid through the delivery lines 11A, 11B from the infusion devices 1A, 1B and to discharge a received fluid to an outlet delivery line 3 connected at an end 30 to an outlet 22 of the relay device 2. The outlet delivery line 3 may, for example, be connected to a patient P by means of a suitable injection needle or the like such that via the outlet delivery line 3 a fluid can be administered to the patient P.

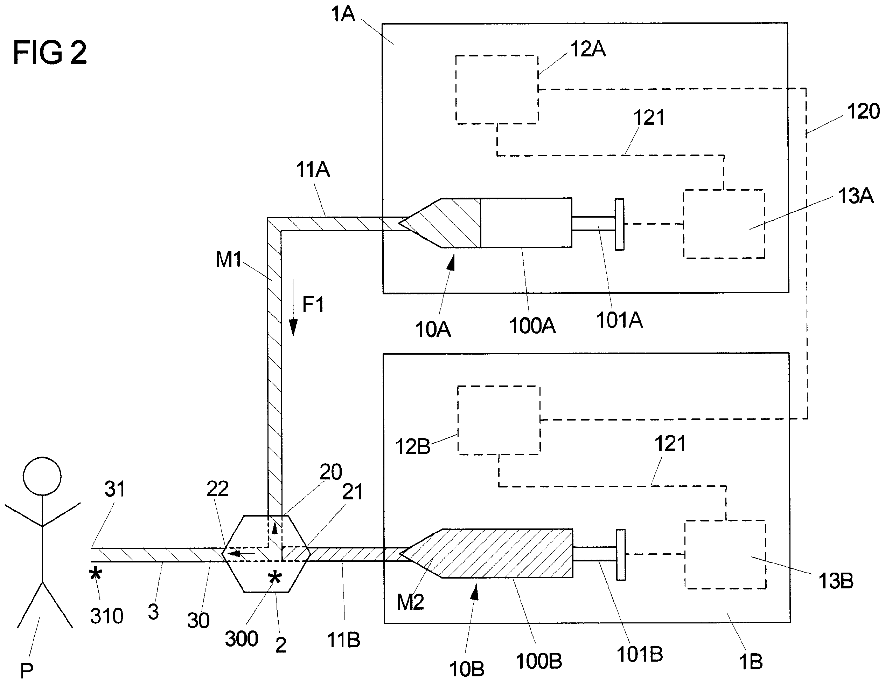

The (continuous) infusion process starts, for example, in a first infusion phase with the first infusion device 1A delivering a fluid, in particular, a medication, from its medication container 100A via the delivery line 11A at a first flow rate F1. As illustrated in Fig. 2, the relay device 2 in this case lets the fluid M1 pass from the inlet 20 to the outlet 22 and hence provides a fluid connection between the delivery line 11A and the outlet delivery line 3 such that the fluid M1 is delivered to the patient P at the first flow rate F1.

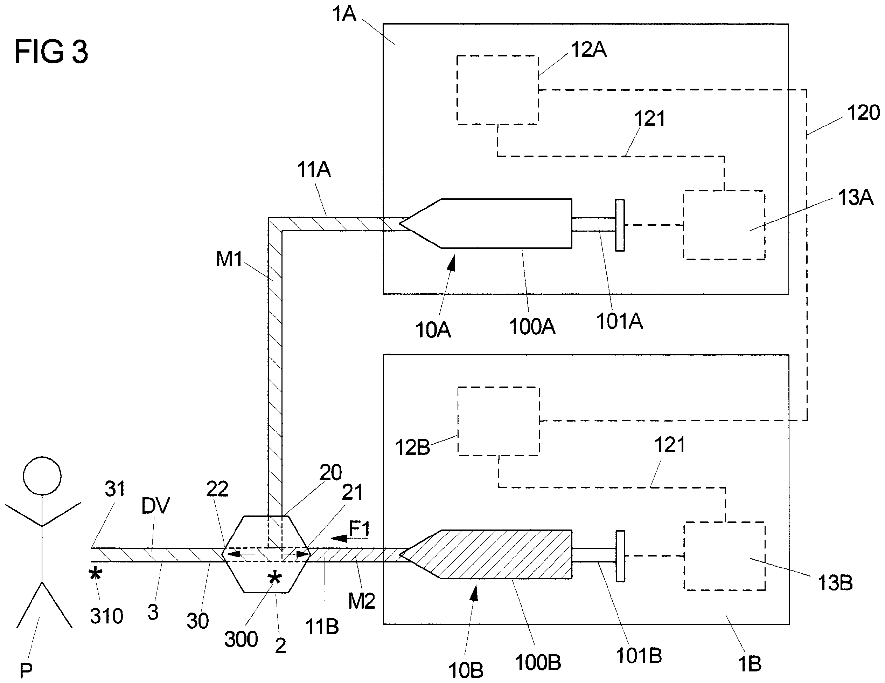

Once the medication container 100A is depleted or nearly depleted, the infusion process shall be switched over to the second infusion device 1B. To control this switching process, both the first infusion device 1A and the second infusion device 1B each comprise a processor 12A, 12B, which are via a communication line 120 in communication with each other. Furthermore, the processors 12A, 12B are, via communication lines 121, in communication with the electric drive device 13A, 13B of the associated infusion device 1A, 1B and furthermore, via communication lines 122 (see Fig. 1), in connection with the relay device 2. At the end of the first infusion phase (when the first medication container 100A is depleted or nearly depleted) a control signal is issued by for example the processor 12A associated with the first infusion device 1A and communicated to the processor 12B of the second infusion device 1B, the control signal causing the operation of the first infusion device 1A to stop and at the same time the operation of the second infusion device 1B to start. At the same time, also the relay device 2 is switched (for example by switching a suitable flow switching means contained in the relay device 2) such that, now, a fluid connection between the inlet 21 and the outlet 22 is obtained, as this is illustrated in Fig. 3.

As is visible from Fig. 3, at the time the infusion process is switched over to the second infusion device 1B, a residual amount of the first fluid M1 delivered by the first infusion device 1A is still present in the outlet delivery line 3, more specifically between the two locations marked with the asterisks 300, 310 at the branch-off point within the relay device 2 on the one hand and the end 31 of the outlet delivery line 3 close to the patient P on the other hand. Upon start of the infusion operation of the second infusion device 1B, this residual volume of the first fluid M1 will be administered to the patient P prior to the second fluid M2 from the second medication container 100B associated with the second infusion device 1B entering into the outlet delivery line 3 and arriving at the patient P.

The first fluid M1 and the second fluid M2 may comprise the same medication, but, for example, at a different concentration. In order to ensure a constant dose rate, therefore, the first flow rate F1 of the first infusion device 1A and the second flow rate F2 of the second infusion device 1B differ. This can be expressed mathematically as follows:

$$\text{dose rate 1 (mg/h)} = \text{flow rate 1 (ml/h)} * \text{concentration 1 (mg/ml)}$$

$$\text{dose rate 2 (mg/h)} = \text{flow rate 2 (ml/h)} * \text{concentration 2 (mg/ml)}.$$

If the first dose rate and the second dose rate shall be equal (dose rate 1 (mg/h) = dose rate 2 (mg/h)), this means that the second flow rate is set as follows:

$$\text{flow rate 2 (ml/h)} = \text{flow rate 1 (ml/h)} * \text{concentration 1 (mg/ml)} / \text{concentration 2 (mg/ml)}.$$

If, for example, the concentration of medication in the first fluid M1 is twice the concentration of medication in the second fluid M2, the second flow rate F2 is to be chosen to be twice as large as the first flow rate F1 in order to obtain a constant dose rate.

However, if the infusion operation by means of the second infusion device 1B would be initiated with the second flow rate F2 immediately upon switching over the infusion process from the first infusion device 1A to the second infusion device 1B, this would lead to the residual volume of the first fluid M1 remaining in the outlet delivery line 3 to be administered to the patient P at the second flow rate F2, which would lead to a change in the dose rate (a peak or a drop in the dose rate), which may have disadvantageous effects on the patient P.

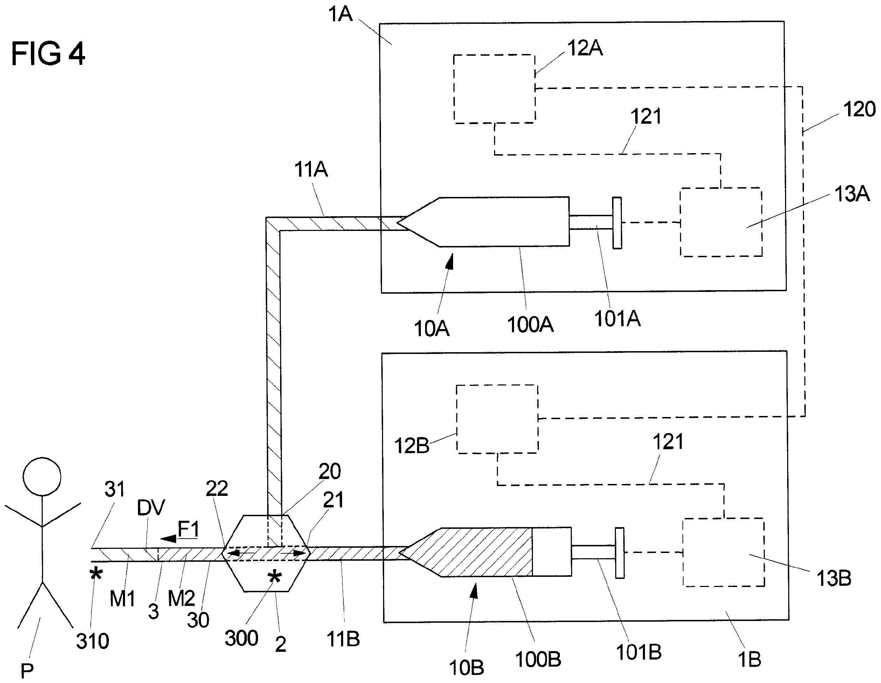

Therefore, when switching over the infusion process from the first infusion device 1A to the second infusion device 1B, in an intermediate phase the second infusion device 1B is operated to run at the first flow rate F1 for a predetermined delivery time, as it is indicated in Fig. 3 and 4. The predetermined delivery time herein is determined by the delivery volume DV of the outlet delivery line 3 (including the flow path within the relay device 2 between the asterisk 300 and the outlet 22) and the first flow rate F1. Namely, the predetermined delivery time equals the delivery volume DV divided by the first flow rate F1:

$$T \text{ (h)} = \text{delivery volume DV (ml) / flow rate 1 (ml/h)}$$

$$= \text{delivery volume DV (ml) * concentration 1 (mg/ml) / dose rate 1 (mg/h)}$$

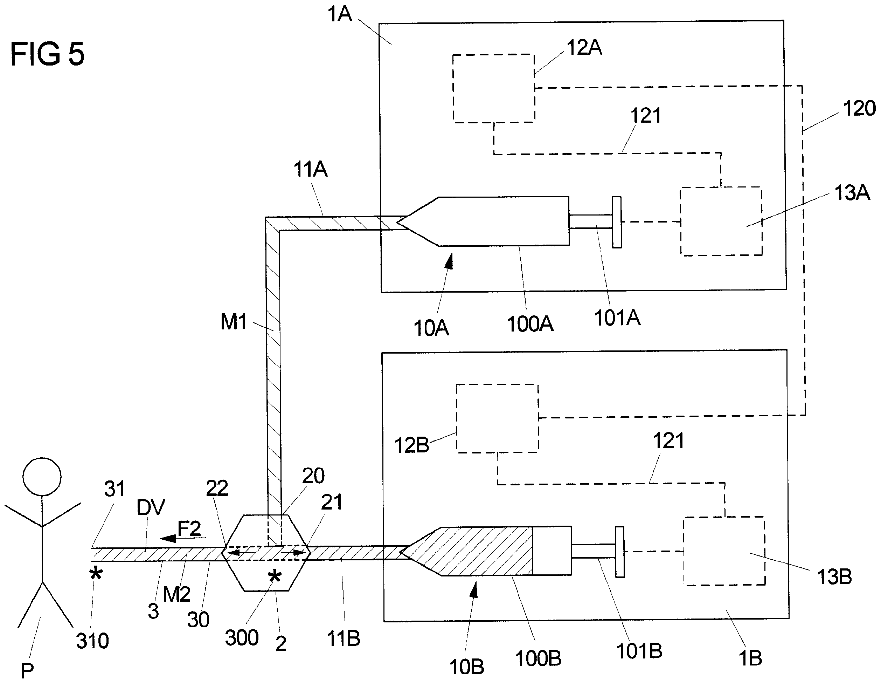

After the second infusion device 1B has been run for the predetermined delivery time T at the first flow rate F1, it is switched to the second flow rate F2, as indicated in Fig. 5. After lapse of the predetermined delivery time T the outlet delivery line 3 is depleted from the first fluid M1 stemming from the first medication container 100A associated with the first infusion device 1A and is filled with the second fluid M2 stemming from the second medication container 100B associated with the second infusion device 1B. The infusion process is then continued at the second flow rate F2, until the second medication container 100B is depleted or nearly depleted, upon which it can be switched over to another, third infusion device or it can be switched back to the first infusion device 1A whose medication container 100a has been refilled or replaced.

By means of the instant method and the instant system it can be assured that a dead volume of residual fluid in the outlet delivery line 3 is not delivered to the patient P at an increased or decreased flow rate differing from the flow rate by which the fluid should be delivered. Hence, a continuous infusion at a constant, unchanged flow rate can be ensured even when switching between different infusion devices for infusing different medicational fluids.

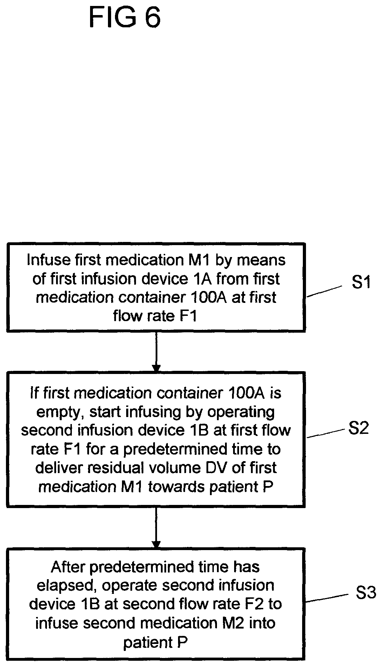

The method of the instant invention is summarized in the flow chart of Fig. 6.

Namely, in a first step S1 a first medication M1 is infused to a patient by means of a first infusion device 1A from a first medication container 100A at a first flow rate F1.

Upon depletion or near depletion of the first medication container 100A, the infusion operation of the first infusion device 1A is stopped and at the same time the infusion operation of the second infusion device 1B is started, which however, at first, takes place at the first flow rate F1 at which the first infusion device 1A was run. This intermediate phase lasts for a predetermined time which is necessary to deliver a residual volume DV remaining in the outlet delivery line 3 towards the patient P (step S2).

After lapse of the predetermined delivery time the second infusion device 1B is switched to the second flow rate F2 and a second fluid from a second medication container 100B of the second infusion device 1B is delivered to the patient P at the second flow rate F2 (step S3).

The invention in principle is not limited to the use of a syringe pump, but can make use also of other infusion devices such as peristaltic (volumetric) infusion devices.

**List of Reference Numerals**

| | |
|---|---|
| 1A, 1B | Infusion device |
| 10A, 10B | Pump device |
| 100A, 100B | Medication container (cylindrical tube) |
| 101A, 101B | Actuation device (plunger) |
| 11A, 11B | Delivery line (tube set) |
| 12A, 12B | Processor |
| 120-122 | Communication line |
| 13A, 13B | Drive device |
| 2 | Relay device |
| 20,21 | Inlet |
| 22 | Outlet |
| 3 | Outlet delivery line |
| 30,31 | End |
| 300, 310 | Asterisk |

DV          Delivery volume
F1, F2      Flow rate
M1, M2      Medication
P           Patient
S1-S3       Method steps


**Claims**

1.  A system for performing a continuous infusion process using at least two infusion devices (1A, 1B), the system comprising:

    a first infusion device (1A) constituted to deliver a first fluid (M1) from a first medication container (100A) through a first delivery line (11A) at a first flow rate (F1) in a first phase,
    a second infusion device (1B) constituted to deliver a second fluid (M2) from a second medication container (100B) through a second delivery line (11B) at a second flow rate (F2) in a second phase following the first phase, and
    a relay device (2) connected to said first delivery line (11A) and said second delivery line (11B) and constituted to receive the first fluid (M1) via the first delivery line (11A) in the first phase and the second fluid (M2) via the second delivery line (11B) in the second phase, wherein the relay device (2) is further constituted to discharge the received first or second fluid (M1, M2) to an outlet delivery line (3) connected to the relay device (2) for administration to a patient (P), the outlet delivery line (3) having a predefined delivery volume (DV),
    wherein the system is constituted such that, subsequent to the delivery of the first fluid (M1) using the first infusion device (1A) in the first phase, the second infusion device (1B) in an intermediate phase is operated for a predetermined delivery time to deliver the second fluid (M2) at the first flow rate (F1), the predetermined delivery time being determined taking the predefined delivery volume (DV) of the outlet delivery line (3) into account, **characterized in that** the first infusion device (1A) and the second infusion device (1B) are operative to communicate with each other to transfer information concerning the first flow rate (F1), wherein the second infusion device (1B) is operative to determine said predefined delivery time according to the first flow rate (F1) and the delivery volume (DV) and wherein one infusion device (1A, 1B) knows about the operational status of the other infusion device (1A, 1B).

2.  The system according to claim 1, **characterized in that** the first infusion device (1A) and the second infusion device (1B) each comprise a processor (12A, 12B), wherein the first infusion device (1A) and the second infusion device (1B) are in communication connection with each other for controlling the operation of the second infusion device (1A) in dependence of the operation of the first infusion device (16).

3.  The system according to claim 1 or 2, **characterized in that** the first infusion device (1A) and the second infusion device (1B) are operative to communicate with each other to transfer information concerning

    - the second flow rate (F2),
    - the filling level of the first medication container (100A),
    - the filling level of the second medication container (100B),
    - the start or stop of an infusion operation,
    - the type of fluid delivered during the first phase, and/or
    - the type of fluid delivered during the second phase.

4.  The system according to one of claims 1 to 3, **characterized in that** the first infusion device (1A) and/or the second infusion device (1B) are constituted as a syringe pump.

5.  The system according to one of claims 1 to 4, **characterized in that** the first medication container (100A) and/or the second medication container (100B) are constituted by a cylindrical tube of a syringe.


**Patentansprüche**

1.  System zum Durchführen eines kontinuierlichen Infusionsprozesses unter Verwendung von mindestens zwei Infusionsvorrichtungen (1A, 1B), wobei das System umfasst:

eine erste Infusionsvorrichtung (1A), die so beschaffen ist, dass sie in einer ersten Phase ein erstes Fluid (M1) aus einem ersten Medikamentenbehälter (100A) durch eine erste Abgabeleitung (11A) mit einer ersten Durchflussrate (F1) abgibt,

eine zweite Infusionsvorrichtung (1B), die so beschaffen ist, dass sie in einer auf die erste Phase folgenden zweiten Phase ein zweites Fluid (M2) aus einem zweiten Medikamentenbehälter (100B) durch eine zweite Abgabeleitung (11B) mit einer zweiten Durchflussrate (F2) abgibt, und

eine Weitergabevorrichtung (2), die mit der ersten Abgabeleitung (11 A) und der zweiten Abgabeleitung (11B) verbunden und so beschaffen ist, dass sie in der ersten Phase das erste Fluid (M1) über die erste Abgabeleitung (11 A) aufnimmt und in der zweiten Phase das zweite Fluid (M2) über die zweite Abgabeleitung (11B) aufnimmt, wobei die Weitergabevorrichtung (2) ferner so beschaffen ist, dass sie das aufgenommene erste oder zweite Fluid (M1, M2) an eine damit verbundene Auslassabgabeleitung (3) abführt, die mit der Weitergabevorrichtung (2) zur Verabreichung an einen Patienten (P) verbunden ist, wobei die Auslassabgabeleitung (3) ein vordefiniertes Abgabevolumen (delivery volume, DV) aufweist, wobei

das System so beschaffen ist, dass im Anschluss an die Abgabe des ersten Fluids (M1) unter Verwendung der ersten Infusionsvorrichtung (1A) in der ersten Phase die zweite Infusionsvorrichtung (1B) in einer Zwischenphase während einer vorgegebenen Abgabezeit betrieben wird, um das zweite Fluid (M2) mit der ersten Durchflussrate (F1) abzugeben, wobei die vorgegebene Abgabezeit unter Berücksichtigung des vordefinierten Abgabevolumens (DV) der Auslassabgabeleitung (3) ermittelt wird, **dadurch gekennzeichnet, dass** die erste Infusionsvorrichtung (1A) und die zweite Infusionsvorrichtung (1B) zum Kommunizieren miteinander funktionsfähig sind, um Informationen bezüglich der ersten Durchflussrate (F1) zu übertragen, wobei die zweite Infusionsvorrichtung (1B) zum Ermitteln der vordefinierten Abgabezeit gemäß der ersten Durchflussrate (F1) und dem Abgabevolumen (DV) funktionsfähig ist und wobei eine Infusionsvorrichtung (1A, 1B) den Betriebsstatus des anderen Infusionsvorrichtung (1A, 1B) kennt.

2. Das System nach Anspruch 1, **dadurch gekennzeichnet, dass** die erste Infusionsvorrichtung (1A) und die zweite Infusionsvorrichtung (1B) jeweils einen Prozessor (12A, 12B) umfassen, wobei die erste Infusionsvorrichtung (1A) und die zweite Infusionsvorrichtung (1B) miteinander in Kommunikationsverbindung zum Steuern des Betriebs der zweiten Infusionsvorrichtung (1A) in Abhängigkeit von dem Betrieb der ersten Infusionsvorrichtung (16) stehen.

3. Das System nach Anspruch 1 oder 2, **dadurch gekennzeichnet, dass** die erste Infusionsvorrichtung (1A) und die zweite Infusionsvorrichtung (1B) dazu dienen, miteinander zu kommunizieren, um Informationen zu übertragen bezüglich

   - der zweiten Durchflussrate (F2),
   - des Füllstands des ersten Medikamentenbehälters (100A),
   - des Füllstands des zweiten Medikamentenbehälters (100B),
   - des Beginns oder Stopps eines Infusionsvorgangs,
   - der Art der während der ersten Phase abgegebenen Flüssigkeit und/oder
   - der Art der während der zweiten Phase abgegebenen Flüssigkeit.

4. System nach einem der Ansprüche 1 bis 3, **dadurch gekennzeichnet, dass** die erste Infusionsvorrichtung (1A) und/oder die zweite Infusionsvorrichtung (1B) als Spritzenpumpe ausgebildet sind.

5. Das System nach einem der Ansprüche 1 bis 4, **dadurch gekennzeichnet, dass** der erste Medikamentenbehälter (100A) und/oder der zweite Medikamentenbehälter (100B) aus einem zylindrischen Rohr einer Spritze bestehen.

## Revendications

1. Système destiné à la réalisation d'un processus de perfusion continue à l'aide d'au moins deux dispositifs de perfusion (1A, 1B), le système comprenant :

   un premier dispositif de perfusion (1A) constitué pour la distribution d'un premier fluide (M1) à partir d'un premier récipient de médicament (100A) à travers une première ligne de distribution (11A) à un premier débit (F1) dans une première phase,
   un second dispositif de perfusion (1B) constitué pour la distribution d'un second fluide (M2) à partir d'un second récipient de médicament (100B) à travers une seconde ligne de distribution (11B) à un second débit (F2) dans une seconde phase suivant la première phase, et
un dispositif relais (2) relié à ladite première ligne de distribution (11 A) et à ladite seconde ligne de distribution (11B) et constitué pour la réception du premier fluide (M1) par l'intermédiaire de la première ligne de distribution (11 A) dans la première phase et le second fluide (M2) par l'intermédiaire de la seconde ligne de distribution (11B) dans la seconde phase, dans lequel le dispositif relais (2) est en outre constitué pour la décharge du premier ou du second fluide reçu (M1, M2) vers une ligne de distribution de sortie (3) reliée au dispositif relais (2) pour l'administration à un patient (P), la ligne de distribution de sortie (3) ayant un volume de distribution prédéfini (DV),
dans lequel

le système est constitué de telle sorte que, à la suite de l'administration du premier fluide (M1) à l'aide du premier dispositif de perfusion (1A) dans la première phase, le second dispositif de perfusion (1B) dans une phase intermédiaire est actionné pendant un temps de distribution prédéterminé pour distribuer le second fluide (M2) au premier débit (F1), le temps de distribution prédéterminé étant déterminé en tenant compte du volume de distribution prédéfini (DV) de la ligne de distribution de sortie (3), **caractérisé en ce que** le premier dispositif de perfusion (1A) et le second dispositif de perfusion (1B) fonctionnent pour communiquer l'un avec l'autre pour le transfert d'informations concernant le premier débit (F1), dans lequel le second dispositif de perfusion (1B) fonctionne pour déterminer ledit temps de distribution prédéfini selon le premier débit (F1) et le volume de distribution (DV) et dans lequel un dispositif de perfusion (1A, 1B) connaît l'état de fonctionnement de l'autre dispositif de perfusion (1A, 1B).

2. Système selon la revendication 1, **caractérisé en ce que** le premier dispositif de perfusion (1A) et le second dispositif de perfusion (1B) comprennent chacun un processeur (12A, 12B), dans lequel le premier dispositif de perfusion (1A) et le second dispositif de perfusion (1B) sont en liaison de communication l'un avec l'autre pour commander le fonctionnement du second dispositif de perfusion (1A) en fonction du fonctionnement du premier dispositif de perfusion (16).

3. Système selon la revendication 1 ou 2, **caractérisé en ce que** le premier dispositif de perfusion (1A) et le second dispositif de perfusion (1B) fonctionnent pour communiquer l'un avec l'autre afin de transférer des informations concernant

- le second débit (F2),
- le niveau de remplissage du premier récipient de médicament (100A),
- le niveau de remplissage du second récipient de médicament (100B),
- le démarrage ou l'arrêt d'une opération de perfusion,
- le type de fluide distribué lors de la première phase, et/ou
- le type de fluide distribué lors de la seconde phase.

4. Système selon l'une des revendications 1 à 3, **caractérisé en** que le premier dispositif de perfusion (1A) et/ou le second dispositif de perfusion (1B) sont constitués sous la forme d'une pompe à seringue.

5. Système selon l'une des revendications 1 à 4, **caractérisé en ce que** le premier récipient de médicament (100A) et/ou le second récipient de médicament (100B) sont constitués par un tube cylindrique d'une seringue.

FIG 1

FIG 2

FIG 3

EP 3 325 048 B1

FIG 4

FIG 5

EP 3 325 048 B1

# FIG 6

```
┌─────────────────────────────────┐
│  Infuse first medication M1 by means │
│  of first infusion device 1A from first │      S1
│  medication container 100A at first │
│         flow rate F1              │
└─────────────────────────────────┘
                 │
                 ▼
┌─────────────────────────────────┐
│  If first medication container 100A is │
│  empty, start infusing by operating │
│  second infusion device 1B at first flow │    S2
│  rate F1 for a predetermined time to │
│  deliver residual volume DV of first │
│  medication M1 towards patient P  │
└─────────────────────────────────┘
                 │
                 ▼
┌─────────────────────────────────┐
│  After predetermined time has     │
│  elapsed, operate second infusion │
│  device 1B at second flow rate F2 to │    S3
│  infuse second medication M2 into │
│         patient P                 │
└─────────────────────────────────┘
```

**EP 3 325 048 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20080269678 A1
- US 20110087189 A1
- US 20130218080 A1
- WO 2005084273 A2
- US 2006224140 A1
- WO 2009149367 A2